# EUROPEAN PATENT APPLICATION

(11) **EP 4 656 622 A1**
(43) Date of publication of application: **03.12.2025**
(21) Application number: 25179710.6
(22) Date of filing: 29.05.2025
(51) Int. Cl.: C05C 5/02, C05F 11/08, C05G 5/27

(54) **NANOFERTILIZER ENRICHED WITH MICRO-ORGANISMS, METHOD OF OBTAINING THE FERTILIZER COMPOSITION, USE OF THE FERTILIZER COMPOSITION AND RHODOTORULA MUCILAGINOSA YEAST**

(30) Priority: 29.05.2024 PL 44871124
(71) Applicant: Uniwersytet Jagiellonski, 31-007 Krakow (PL)
(72) Inventor: Turnau, Katarzyna, 30-095 Kraków (PL); Zapotoczny, Szczepan, 30-381 Kraków (PL); Bie -Kostycz, Patrycja, 38-450 Jasionka (PL)
(74) Representative: Witek, Rafal

(57) **Abstract**

A nanofertilizer enriched with microorganisms, a method for obtaining thereof and a *Rhodotorula mucilaginosa* yeast strain contained therein are disclosed. The nanofertilizer of the invention is used in the cultivation of plants, especially vascular, mycorrhizal plants.

## Description

The subject of the invention is a nanofertiliser enriched with micro-organisms, a method of obtaining it and a strain of *Rhodotorula mucilaginosa* yeast contained therein. The nanofertiliser is used in the cultivation of plants especially vascular, mycorrhizal plants.

Mycorrhizal fungi are commonly found in the soil where plants are grown. As a result of the interaction of these organisms (plants and fungi), a mutualistic symbiosis (mutually beneficial for both partners) occurs, which involves the supply of carbohydrates produced by the plant (as a result of photosynthesis) to the mycelium, and minerals and water by the mycelium to the plants. As a result of this symbiosis, plants are potentially better able to thrive and are less sensitive to drought and pathogens. The abundant development of mycelium in the soil prevents soil erosion, stimulates micro-organism diversity in the soil, and thus can stimulate plant diversity in communities. The use of commercial chemical fertilisers at high doses reduces the occurrence of mycorrhiza or excludes it altogether. The result of this process is the search for new methods to restore soil biodiversity, which, however, cannot be achieved without mycorrhizal fungi.

US patent application US2021139386A1 discloses a combined NPK-Si fertiliser product comprising a mineral NPK fertiliser containing at least one nitrogen, phosphorus or potassium compound and amorphous silicon dioxide in particulate form, the ratio of mineral NPK fertiliser to amorphous silicon dioxide being between 10:90 to 90:10 based on the dry weight of the at least one nitrogen, phosphorus or potassium nutrient compound and the amorphous silicon dioxide contained in the product.

A M. Sc. thesis from 2021, entitled: "Interactions of wheat with Rhodotorula mucilaginosa and related microorganisms", presented the results of a study on interactions between plants and endophytic microorganisms, which could lead to increased crop yields, reduced pesticide use and, in the future, contribute to the large-scale use of endophytes in agriculture. The study investigated interactions between microorganisms and root hairs of wheat *(Triticum aestivum).* Interactions of endophytic yeasts *(Rhodotorula mucilaginosa),* hereafter also described as *Rhodotorula,* bacteria *(Pseudomonas putida)* and pathogenic filamentous fungi (*Pyrenophora teres)* were included. Wheat cultures were established in liquid medium and inoculated with microorganisms. The interactions taking place were observed using light, fluorescence and scanning microscopy. The interactions between the test organisms contribute to the growth and function of the wheat. The results showed that the pathogen development was slowed down in the presence of yeast and bacteria. The yeasts tested show 'predation' characteristics towards the pathogenic fungus, contributing to a reduction in pathogen viability. In the presence of heavy metals and bacteria, *R. mucilaginosa* changed its form of growth from yeast to filamentous form, which may facilitate their colonisation by bacteria.

The scientific publication "Reducing nitrogen dosage in Triticum durum plants with urea-doped nanofertilisers" (Ramírez-Rodríguez G. B. et al. 2020, Nanomaterials 10(6), 1043) describes urea-enriched phosphate nanoparticles for fertilising *Triticum durum* plants.

The technical problem would be to provide a fertiliser with reduced NPK leaching from the substrate, which would translate into a reduction in fertiliser use by at least half, enabling the maintenance of a proper soil microbiota, inhibition of algal blooms, as well as protection of the plant from attack by certain soil pathogens. In addition, the fertiliser should also be characterised by increased moisture retention.

The subject of the invention is a nanofertiliser composition, a method of obtaining thereof and a microorganism strain contained therein defined in detail in the appended claims.

The nanofertiliser of the invention belongs to the group of fertilisers with slow availability to plants. It is enriched with an inoculum containing microorganisms. The advantage of the nanofertiliser is the low leachability of nutrients into groundwater and the beneficial effect on plant symbiotic interactions, including with mycorrhizal fungi. It has been tested on wheat, blue honeysuckle (also known as kamchatka berry), narrowleaf plantain, *Berkheya coddii* (mycorrhizal Ni hyperaccumulator) and others. Most of the results were obtained for narrowleaf plantain, which is an indicator plant. It forms arbuscular mycorrhiza with a wide range of mycorrhizal fungi and is a mycorrhizal-dependent plant. The developed nanofertiliser can be used to treat plants capable of forming mycorrhiza (about 80% of vascular plants, including crops except brassicas). Mycorrhizal plants include maize, most cereals, grasses, tomato, lettuce. The above-mentioned mycorrhizal plants do grow after the application of commercial fertilisers, but their susceptibility to soil desiccation and pathogen attack is considerable. They also have a limited amount of aroma and flavour, which reduces their quality. In the case of plants grown especially on a metal-containing substrate (cultivation of *Berkheya coddii,* a plant that hyperaccumulates nickel), a significant improvement was found not only in plant biomass growth but also in the degree of mycorrhization, which supports the development of a proper substrate structure through the expansion of the extra-root mycelium of mycorrhizal fungi. The proposed nanofertiliser is only slightly released into the groundwater because it is difficult to dissolve in water and is made available through the action of microorganisms, in this case fungi (which was verified by watering the test cultures with excess water), while the availability of individual elements (NPK) is secured through the yeast introduced into the substrate and the mycorrhizal fungi present in the substrate. The nanofertiliser is suitable for supporting the growth of mycorrhizal crops in organic farming, allotment gardens, in the cultivation of herbs such as basil or thyme when abandoning the use of peat substrates and replacing it with substrates that allow mycorrhizal growth, thus reducing the need for frequent watering and producing much more aromatic plants. The fertiliser formulation of the invention solves the problem of the long-term and efficient delivery of plant growth-stimulating minerals, reducing fertiliser consumption and the need for frequent delivery. This is accomplished by the simultaneous use of mineral nutrient carriers slowing their release into the soil and fungi (yeast) improving the uptake of these nutrients (released in small amounts but over a longer period of time) by the plants.

The invention is illustrated in the drawing, where:

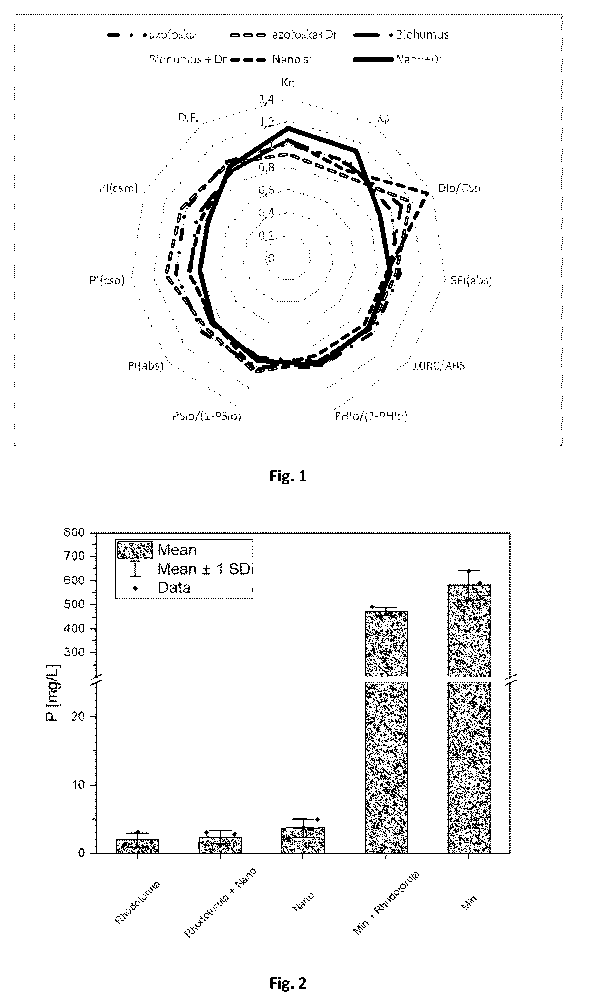

Fig. 1 shows the parameters of the photosynthetic apparatus depending on the applied fertiliser at doses comparable and in accordance with the manufacturers' recommendations; the addition of yeast also shows a positive effect in the case of commercial fertilisers, photosynthetic parameters: RC/ABS - density of reaction centres per chlorophyll; DIO/CSo - thermal dissipation of excitation energy by PSII of the photosynthesising sample; PI(abs) - PSII performance index versus absorption; PI(cs) - PSII performance index versus sample; PSlo/(1-PSlo) - electron transport efficiency beyond Q_{A}⁻; PHIo/(1-PHIo) - primary photochemical reaction efficiency index; Kp - photochemical reaction constant; Kn - non-photochemical reaction constant; DF - driving force index in PSII;
Fig. 2 shows the degree of phosphorus leaching from the substrate during abundant watering in the presence of nanofertiliser (Nano) alone, *Rhodotorula* alone, *Rhodotorula* with Nano and mineral fertilisers (Min), and mineral fertilisers with *Rhodotorula;*

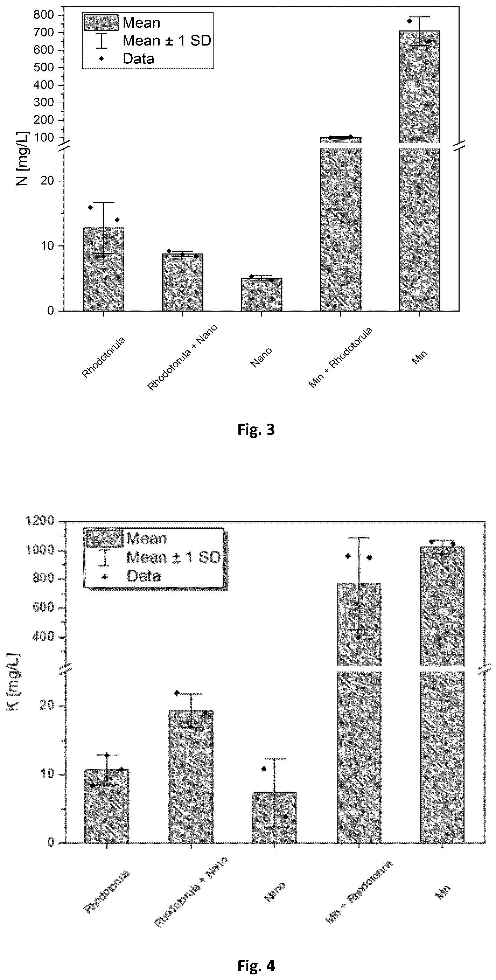

Fig. 3. shows the degree of nitrogen leaching from the substrate during abundant watering in the presence of nanofertilisers (Nano) alone, *Rhodotorula* alone, *Rhodotorula* with Nano and mineral fertilisers (Min), and mineral fertilisers with *Rhodotorula;*
Fig. 4 shows the degree of potassium leaching from the substrate during abundant watering in the presence of only nanofertilisers (Nano) alone, *Rhodotorula* alone, *Rhodotorula* with Nano and mineral fertilisers (Min), and mineral fertilisers with *Rhodotorula;*
Fig. 5 shows the leaf biomass (in grams) per pot in crops treated with different doses of nanofertilisers in the presence or absence of yeast (X+0); different letters above the bars indicate no statistically significant difference; sums below the bars indicate the amount of g of nanofertiliser added with what amount by microorganisms,

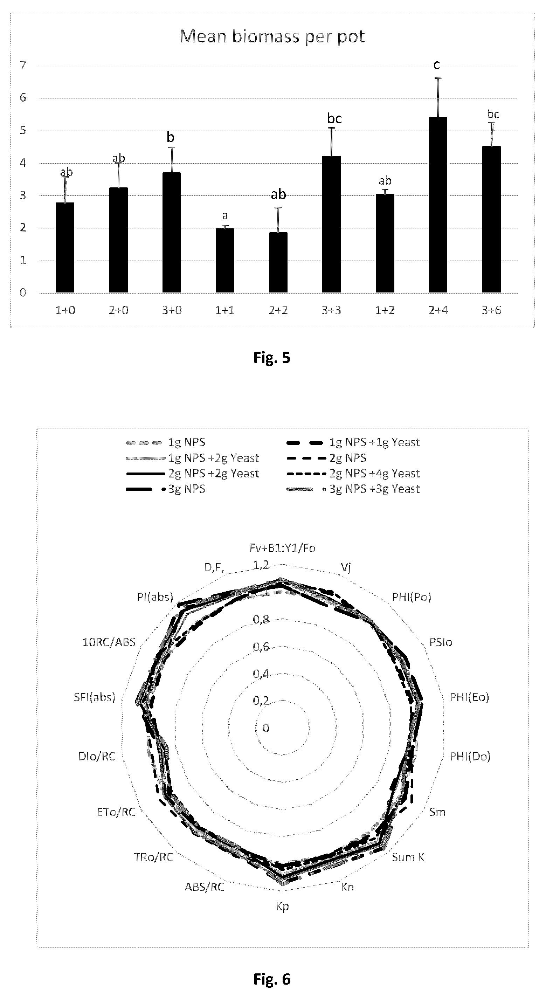

Fig. 6. Shows the parameters of the photosynthetic apparatus as a function of the amount of nanofertiliser (NPS) and the amount of yeast; ABS/RC - energy absorption per single reaction centre; DIO/RC - total dissipation of energy not captured by the reaction centre in the form of heat, fluorescence and transfer to other systems at t=0; PI(abs) - PSII performance index versus absorption; PI(cs) - PSII performance index versus sample; PSlo(Eo) - quantum yield of electron transport; PHIo(Do) - quantum yield of energy dissipation; Kp - photochemical reaction constant; Kn - non-photochemical reaction constant; DF - driving force index in PSII; ETo/RC - electron transport rate through one reaction centre; Sm - normalised total area over the OJIP curve; SFl(abs) - structure-function index reporting the strength of the internal drivers of the PSII reaction; Sum K=Kp+Kn;
Figs 7a-7c show respectively: 7a - mycorrhizal frequency (F%), 7b - degree of mycorrhizal colonisation (M%) and 7c - abundance of arbuscules (A%) in crops treated with different doses of nanofertilisers (Xg+ Yg) in the presence or absence of yeast (X+0); different letters above the bars indicate the presence of a statistically significant difference.
Fig. 8 shows the ability to maintain moisture in samples with nanofertilisers (NPS) and fertilisers with added yeast (NPS Y); different letters above the bars indicate the presence of a statistically significant difference;
Fig. 9 shows particle sizes based on dynamic light scattering: sample M chemicals alone, sample S after sonication, sample Rh after addition of microbial consortium, sample Zn after addition of zinc; whiskers show standard deviations;
Figs 10a-10e. Roots of narrowleaf plantain after application of nanofertilisers with the addition of yeast.

### Example 1

The preparation of nanofertilizers was based on the modified procedure described in "Reducing nitrogen dosage in Triticum durum plants with urea-doped nanofertilizers" (Ramírez-Rodríguez G. B. et al. 2020, Nanomaterials 10(6), 1043). Nanofertilisers were prepared by mixing two solutions of 100 mL each. Solution A contained: 3.28 g (0.02 mole) of calcium nitrate Ca(NO₃)₂, 5.16 g (0.02 mole) of sodium citrate (Na₃Cit), 8 g (0.13 mole) of urea CO(NH₂)₂ and 0.75 g (0.003 mole) of zinc nitrate Zn(NO₃)₂.

Solution B contained: 0.012 mole of K₂HPO₄, 0.01 mole of sodium carbonate Na₂CO₃. The two solutions were combined so that they met in a common stream as they were poured into the container. The solution was then stirred using a magnetic stirrer for about 15 minutes at room temperature. The combination of the two solutions resulted in the formation of nanoparticle-like compounds that are difficult to dissolve in water.

Then, 100 cm³ of a suspension (1x10⁹ CFU/mL) containing the yeast *Rhodotorula mucilaginosa* strain 471 (isolated from *Plantago major* plantain collected from a copper heap site in Norway) was added to the above mixture. The selection of strain 471 was based on a screening study of various strains held in the collection maintained by the authors. As part of the screening performed, the pre-selected strains of symbiotic microorganisms were compared in terms of their ability to stimulate growth, the state of the photosynthetic apparatus (using Handy PEA) and the efficiency of nitrogen uptake by narrowleaf plantain and wheat, which was an important part of the selection procedure used.

Unexpectedly, the yeast *Rhodotorula mucilaginosa* strain 471 showed the most favourable combination of the key functional traits tested during screening.

The yeast *Rhodotorula mucilaginosa* strain 471 was deposited in accordance with the Budapest Treaty on April 2^{nd}, 2024 in the Culture Collection of Industrial Microorganisms maintained at the Prof. Wactaw D 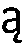 browski Institute of Agricultural and Food Biotechnology - State Research Institute (Prof. Wactaw D 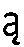 browski IAFB-SRI) under the accession number KKP 2100p.

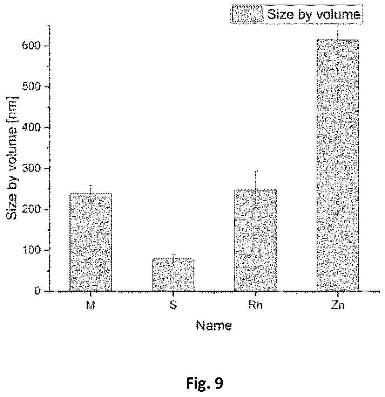

The suspension of strain 471 was transferred to centrifuge tubes and centrifuged (15 min, 5000 rpm). The supernatant was removed, the tubes were refilled with deionised water, and the pellet was mixed appropriately until a turbid suspension was obtained (as prior to the first centrifugation). Washing was repeated twice. The solution was poured off and the residue was placed in a dish and left at 40-50°C to dry. The fertiliser thus prepared is a low-cost substitute for commercially-available fertilisers designed to grow plants that form symbioses with mycorrhizal fungi. The nanoparticles obtained are in the range 30-600 nm. Particle size was determined by dynamic light scattering. Fig. 9 shows a comparison of the average size 'by volume', where:
- for the sample M, containing only chemical components, size-homogeneous nanoparticles with diameters of approx. 240 nm were found. The results indicate a small (preferably) size spread;
- for the sample S, subjected to sonication, small particles were found, but the sonication process breaks them into two fractions: very small around 30-40 nm and larger around 200-300 nm. This is preferable as differentiation of micronutrient release is achieved;
- for the sample Rh, containing microorganisms and chemical components, relatively homogeneous particle sizes were found, similar to sample M;
- for the sample Zn - when Zn was added, it was found that it could contain fractions of different sizes. There are very small nanoparticles, but at the same time there are aggregates of much larger sizes.

The grain size of the nanofertiliser is important because larger particles, e.g. of micrometre size, would release the minerals too slowly. Smaller nanoparticles or free ions mean too rapid release and delivery of these nutrients to the plants (short-lived delivery). Thus, the unfavourable time profile of mineral release would limit the growth effect in the plants. Therefore, preferably the nanoparticles should be of the size indicated above, with a size of approximately 100 nm being most preferred.

### Example 2

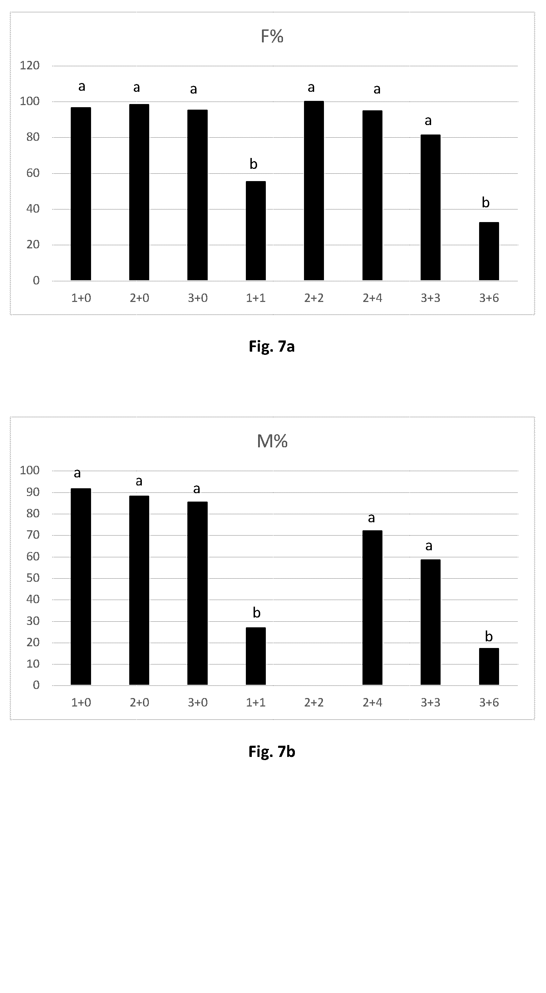

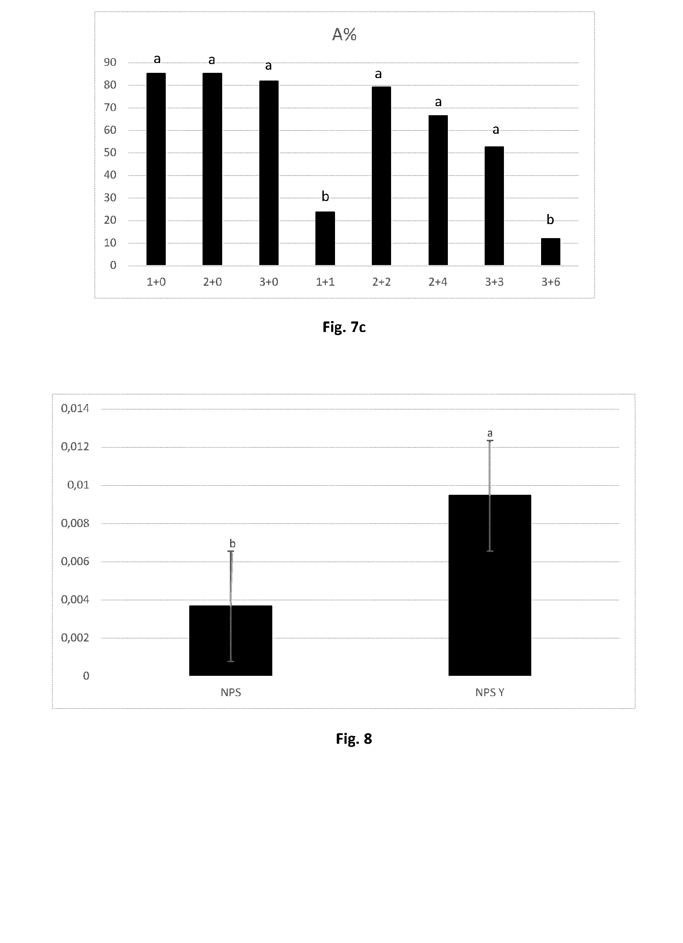

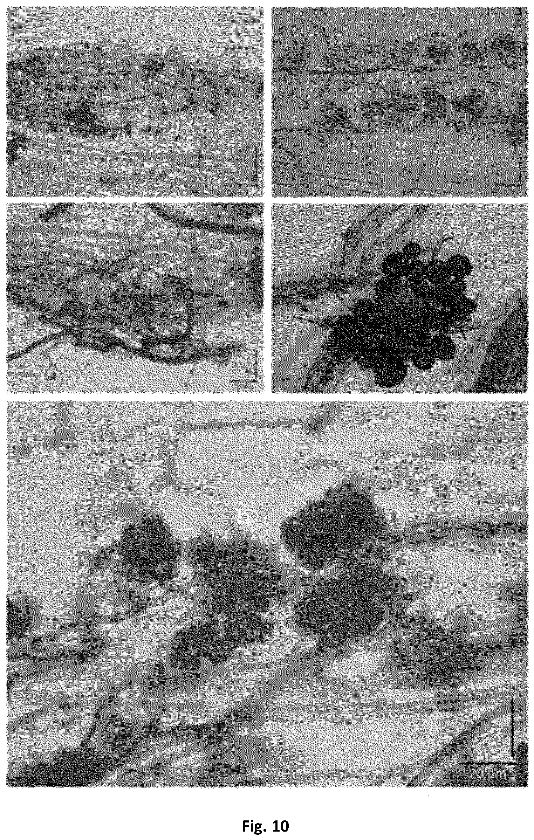

The prepared nanofertiliser was tested under culture chamber conditions. The experiment was carried out five times, with five replicates each time. The results were confirmed and compared with commercially available fertilisers. Measurements of photosynthetic apparatus stress using Handy PEA and nutrient loss due to watering were taken. After cultivation, the plants were harvested, the above-ground parts were left to dry and then weighed. The underground parts were digested in 10% KOH, washed, acidified with 5% lactic acid and stained with aniline blue in 5% lactic acid. After preparation of microscope slides, the degree of mycorrhization, the abundance of arbuscules and the frequency of mycorrhiza were assessed (Figs 7a-7c). Figs 10a-10e show characteristic mycorrhizal structures proving the ability to form mycorrhiza after fertiliser application. In the control samples at this amount of commercial fertiliser, the roots are devoid of mycorrhizal fungi.

Studies on the obtained preparation were carried out with a total of more than 200 pots in the presence of mycorrhizal fungi in a substrate consisting of sand and volcanic clay. Narrowleaf plantain (a plant that responds strongly to the degree of mycorrhization) was used as a model plant. In the first stage of the study, the growth of the plantain was compared using several types of commercial fertiliser and a nanofertiliser produced according to the invention. Based on chlorophyll fluorescence assays (HANDY PEA), there were no statistically significant differences between the functioning of the photosynthetic apparatus and in plant biomass between the preparations used (Fig. 6). However, there was a difference in the abundance of symbiotic mycorrhizal fungi and in elemental efflux during abundant watering.

In conclusion, the developed nanofertiliser contains inorganic nanoparticles and a selected fungal strain *Rhodotorula mucilaginosa* 471. The bionanofertiliser should reduce fertiliser use by at least half, promotes the maintenance of a proper soil microbiota, does not contribute to algal blooms, reduces water requirements (Fig. 8) and can protect plants from attack by certain soil pathogens.

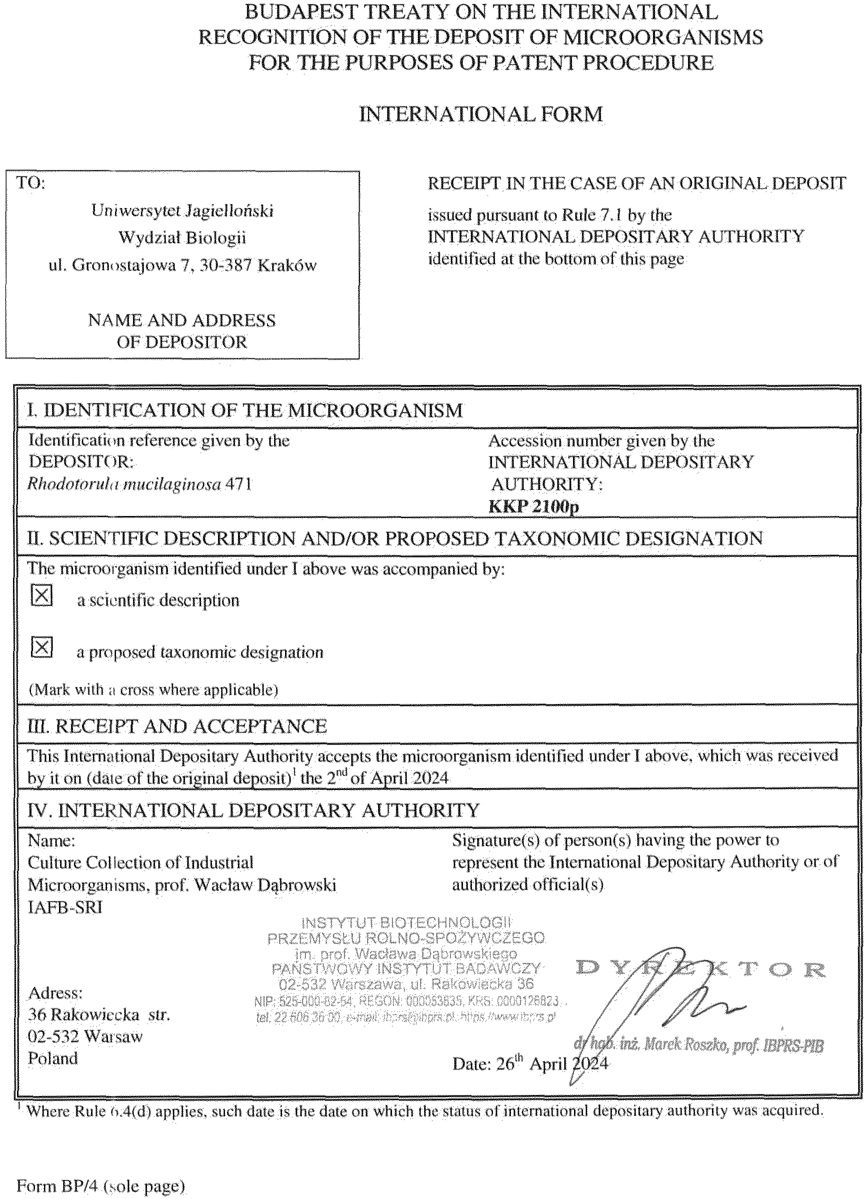

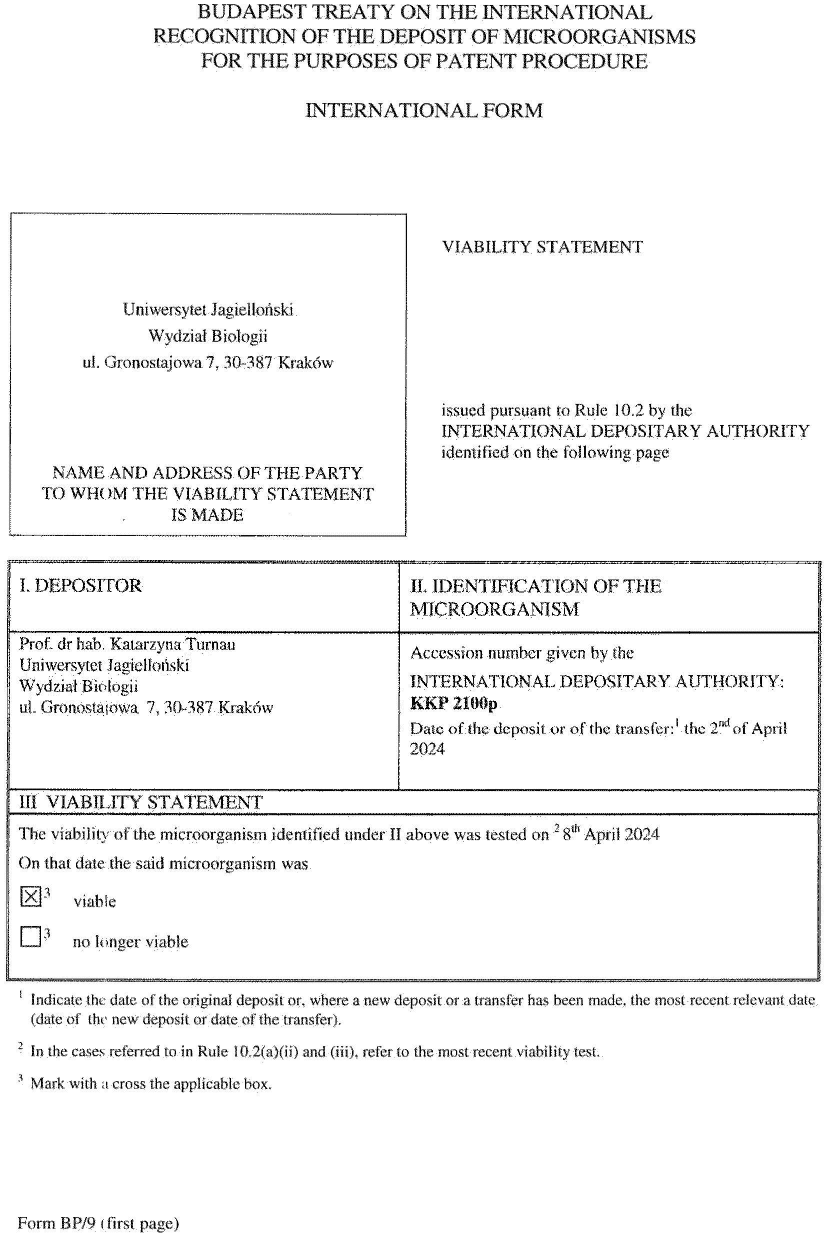

## Claims

1. A fertilizer composition containing microorganisms, **characterized in that** it contains an inorganic part, wherein the inorganic part is in the form of nanoparticles with a diameter of 30 nm to 600 nm, and an organic part, which is the yeast *Rhodotorula mucilaginosa* strain 471 deposited in the Culture Collection of Industrial Microorganisms maintained at the Prof. Wactaw D 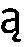 browski IAFB-SRI under the accession number KKP 2100p, wherein the inorganic part constitutes a carrier of the organic part.

2. The composition of claim 1, **characterised in that** the inorganic part is obtained by combining a solution containing in 100 cm³ from 0.015 mole to 0.025 mole of calcium nitrate, from 0.015 mole to 0.025 mole of sodium citrate, from 0.1 mole to 0.2 mole of urea, from 0.001 mole to 0.004 mole of zinc nitrate with an equal volume of a solution containing in 100 cm³ from 0.01 mole to 0.02 mole of potassium hydrogen phosphate and from 0.01 mole to 0.02 mole of sodium carbonate.

3. The composition of claim 2, **characterised in that** the inorganic part is obtained by combining a solution containing in 100 cm³ from 0.02 mole to 0.025 mole of calcium nitrate, preferably 0.02 mole, from 0.02 mole to 0.025 mole of sodium citrate, preferably 0.02 mole, from 0.1 mole to 0.15 mole of urea, preferably 0.13 mole, from 0.003 mole to 0.004 mole of zinc nitrate, preferably 0.003 mole, with a solution containing in 100 cm³ from 0.01 mole to 0.015 mole of potassium hydrogen phosphate, preferably 0.012 mole, and from 0.01 mole to 0.015 mole of sodium carbonate, preferably 0.01 mole.

4. A method for obtaining a microorganism-containing fertilizer composition comprising:
a) obtaining a mixture of inorganic ingredients,
b) combining the mixture of inorganic ingredients with microorganisms,
c) purifying the suspension of the mixture of inorganic ingredients and microorganisms,
**characterised in that**
in step a) the mixture of inorganic constituents is obtained by combining a solution containing in 100 cm³ from 0.015 mole to 0.025 mole of calcium nitrate, from 0.015 mole to 0.025 mole of sodium citrate, from 0.1 mole to 0.2 mole of urea, 0.001 mole to 0.004 mole of zinc nitrate with a solution containing in 100 cm³ from 0.01 mole to 0.02 mole of potassium hydrogen phosphate and from 0.01 mole to 0.02 mole of sodium carbonate, followed by separation of the mixture of solid components of the suspension obtained,
in step b) the mixture from step a) is combined with a suspension of microorganisms containing the yeast *Rhodotorula mucilaginosa* strain 471 deposited in the Culture Collection of Industrial Microorganisms maintained at the Prof. Wactaw D 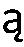 browski IAFB-SRI under the accession number KKP 2100p,
and in step c) the solid components of the resulting mixture forming the fertiliser composition are isolated.

5. The method of claim 4, **characterised in that** the mixture of inorganic components is obtained by combining a solution containing in 100 cm³ from 0.02 mole to 0.025 mole of calcium nitrate, preferably 0.02 mole, from 0.02 mole to 0.025 mole of sodium citrate, preferably 0.02 mole, from 0.1 mole to 0.15 mole of urea, preferably 0.13 mole, from 0.003 mole to 0.004 mole of zinc nitrate, preferably 0.003 mole, with a solution containing in 100 cm³ from 0.01 mole to 0.015 mole of potassium hydrogen phosphate, preferably 0.012 mole, and from 0.01 mole to 0.015 mole of sodium carbonate, preferably 0.01 mole.

6. The method of claim 4, **characterised in that** the concentration of microorganisms in the suspension used in step b) is 1x10⁹ CFU/mL.

7. The method of claim 4, **characterised in that** the diameter of the fertiliser particles obtained in step c) is between 30 nm and 600 nm.

8. The method of claim 4, **characterised in that** the isolation of solid components introduced in step c) comprises centrifugation, washing twice with deionised water and drying, followed by drying to a constant mass, the drying being carried out at a temperature between 40°C and 50°C.

9. Use of the fertiliser as defined in claims 1-3 or obtained by the method of claims 4-8 for the cultivation of plants, especially vascular and mycorrhizal plants.

10. *Rhodotorula mucilaginosa* yeast strain 471 deposited in the Culture Collection of Industrial Microorganisms maintained at the Prof. Wactaw D 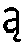 browski IAFB-SRI under accession number KKP 2100p.
